# FASCICULE DE BREVET EUROPEEN

(11) **EP 0 756 864 B1**
(45) Date de publication et mention de la délivrance du brevet: **26.11.1997**
(21) Numéro de dépôt: 96401523.4
(22) Date de dépôt: 10.07.1996
(51) Int. Cl.: A61K 7/48, A61K 7/02, A61K 7/027, A61K 7/025

(54) **Utilisation de l'association de deux huiles particulières dans une composition notamment cosmétique, et composition obtenue**
Verwendung der Assoziation von zwei besonderen Ölen in einer kosmetischen Zubereitung, und die damit enthaltende Zubereitung
Use of the association of two particular oils in a cosmetic composition, and the composition obtained thereby

(30) Priorité: 28.07.1995 FR 9509254
(43) Date de publication de la demande: 05.02.1997
(73) Titulaire: L'OREAL, 75008 Paris (FR)
(72) Inventeur: Agostini, Isabelle, 92290 Chatenay Malabry (FR); Pradier, François, 92260 Fontenay aux Roses (FR); Arnaud, Pascal, 94000 Creteil (FR)
(74) Mandataire: Dodin, Catherine

(56) Documents cités:
- EP-A- 0 197 485
- EP-A- 0 268 950
- EP-A- 0 310 252
- EP-A- 0 602 905
- FR-A- 2 646 346
- FR-A- 2 707 485
- US-A- 5 288 482
- PATENT ABSTRACTS OF JAPAN vol. 010, no. 230 (C-365), 9 août 1986 & JP 61 065808 A (SHISEIDO CO LTD), 4 avril 1986,
- PATENT ABSTRACTS OF JAPAN vol. 012, no. 198 (C-502), 8 juin 1988 & JP 62 298519 A (SHISEIDO CO LTD;OTHERS: 01), 25 décembre 1987,

## Description

La présente invention a trait à une composition notamment cosmétique susceptible d'être appliquée sur la peau et/ou les lèvres, et en particulier une composition anhydre pour le soin et/ou le maquillage de la peau et/ou des lèvres, notamment un rouge à lèvres sous forme de stick, ou un fond de teint.

Les compositions de rouge à lèvres et de fond de teint comprennent généralement des corps gras tels que des huiles, des composés pâteux et des cires, ainsi qu'une phase particulaire généralement composée de charges et de pigments.
Ces compositions, lorsqu'elles sont appliquées sur la peau ou les lèvres, présentent l'inconvénient de transférer, c'est-à-dire de se déposer au moins en partie, en laissant une trace, sur certains supports avec lesquels elles peuvent être mises en contact, et notamment un verre, une tasse, un vêtement ou la peau. Il s'en suit une persistance médiocre du film sur la peau ou sur les lèvres nécessitant de renouveler régulièrement l'application de la composition de fond de teint ou de rouge à lèvres.
Par ailleurs, l'apparition de traces inacceptables sur certains vêtements et notamment sur les cols de chemisier peut écarter certaines femmes à utiliser ce type de maquillage.
Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières. Tous ces phénomènes engendrent un effet inesthétique, que la consommatrice souhaite bien évidemment éviter.

Depuis plusieurs années, de nombreux cosméticiens se sont intéressés aux compositions de rouge à lèvres et plus récemment aux compositions de fond de teint 'sans transfert'. Ainsi, il a été envisagé dans la demande de brevet JP-A-61-65809 des compositions de rouge à lèvres 'sans transfert' contenant 1 à 70% en poids de résine liquide de silicone à motifs répétitifs silicates (ou à réseau tridimensionnel) comportant des chaînes pendantes alkylées de 1 à 6 atomes de carbone ou phénylées, 10 à 98% en poids d'une huile de silicone volatile à chaîne Si-O cyclique et à radicaux méthyle et des charges pulvérulentes. Ces compositions, bien que fort satisfaisante sur la propriété de 'sans transfert' présentent l'inconvénient d'être liquides et donc peu commodes à utiliser, ou tout au moins loin du concept classique d'un rouge à lèvres en bâton, limitant le nombre de femmes susceptibles d'utiliser ce type de rouge à lèvres. De plus, le film obtenu sur les lèvres après évaporation de l'huile de silicone présente l'inconvénient de devenir inconfortable au cours du temps (sensation de dessèchement et de tiraillement, écartant encore un certain nombre de femmes de ce type de rouge à lèvres). Pour améliorer le confort de ce type de composition, on pourrait ajouter des huiles non volatiles siliconées ou non, mais dans ce cas on perd en efficacité 'sans transfert'. De plus, ces compositions sont longues à sécher, c'est-à-dire que le résultat 'sans transfert' n'apparaît qu'au bout de plusieurs minutes.
Plus récemment, il a été envisagé dans la demande de brevet EP-A-602905 des rouges à lèvres 'sans transfert' contenant une silicone volatile cyclique ou linéaire et à chaînes méthylées pendantes et une résine de silicone comportant une chaîne estérifiée pendante ayant au moins 12 atomes de carbone. Le film de rouge à lèvres a encore l'inconvénient de manquer de confort à l'application et notamment d'être trop sec.

La présente invention a pour but de proposer une composition notamment anhydre de soin ou de maquillage permettant de remédier à ces inconvénients et permettant en particulier l'obtention d'un film ne transférant pas, ne migrant pas, ne tachant pas un support avec lequel il serait en contact, tout en présentant des propriétés cosmétiques améliorées par rapport à celles des produits 'sans transfert' de l'art antérieur notamment des propriété de glissant, de non tiraillement et de non dessèchement des lèvres.

Ainsi, un objet de l'invention est l'utilisation de l'association d'une huile volatile et d'une huile siliconée phénylée dans une composition comprenant une phase grasse afin de diminuer le transfert et/ou la migration et/ou d'améliorer la tenue, de ladite composition.
Un autre objet de l'invention est une composition comprenant, dans une phase grasse, une huile volatile, une huile siliconée phénylée et moins de 20% en poids d'huile hydrocarbonée non volatile.
Un autre objet de l'invention est un procédé pour limiter, diminuer et/ou empêcher le transfert d'une composition de maquillage ou de soin de la peau, des muqueuses et/ou des semi-muqueuses, notamment un rouge à lèvres ou un fond de teint, consistant à introduire dans ladite composition une phase grasse comprenant une huile volatile, une huile siliconée phénylée et moins de 20% en poids d'huile hydrocarbonée non volatile.

L'invention s'applique aux compositions à appliquer sur la peau, les semi-muqueuses et/ou les muqueuses, et notamment, non seulement aux produits de maquillage des lèvres mais aussi aux produits de soin des lèvres ainsi qu'aux produits de maquillage et de soin de la peau tels que les fonds de teint. En effet, les produits de maquillage du visage présentent les mêmes inconvénients de 'transfert' sur un support que les rouges à lèvres.
On considère notamment comme muqueuse, la partie interne de la paupière inférieure; parmi les semi-muqueuses, on entend plus particulièrement les lèvres du visage.

La composition de l'invention peut se présenter sous forme solide et par exemple sous forme d'un stick. En outre, elle permet l'obtention d'un film homogène, facilement applicable, s'étalant aisément et uniformément. Le film obtenu présente également une texture légère et reste confortable, non sec, et peut être porté tout au long de la journée.

La composition selon l'invention comprend donc au moins une huile volatile, qui peut être choisie en particulier parmi les huiles hydrocarbonées ou les huiles siliconées, cycliques ou linéaires, seules ou en mélange. Par huile volatile, on entend dans la présente description, toute huile susceptible de s'évaporer au contact de la peau. De préférence, on utilise des huiles dont le point éclair est suffisamment élevé pour permettre l'utilisation de ces huiles en formulation, et suffisamment bas pour obtenir l'effet évanescent souhaité. On emploie de préférence des huiles dont le point éclair est de l'ordre de 40-100°C.
Parmi les huiles siliconées volatiles, on peut citer la cyclotétradiméthylsiloxane, la cyclopentadiméthylsiloxane, la cyclohexadiméthylsiloxane et le méthylhexyldiméthylsiloxane. Parmi les huiles hydrocarbonées volatiles, on peut citer les isoparaffines.
La composition selon l'invention peut comprendre 8-70% en poids, de préférence 30-60%, d'huiles volatiles par rapport au poids total de la composition.

La composition selon l'invention comprend également au moins une huile siliconée phénylée. Cette huile peut être un polyphénylméthylsiloxane ou un phényltriméthicone, ou un mélange de différentes huiles siliconées phénylées, et en particulier peut répondre à la formule suivante : 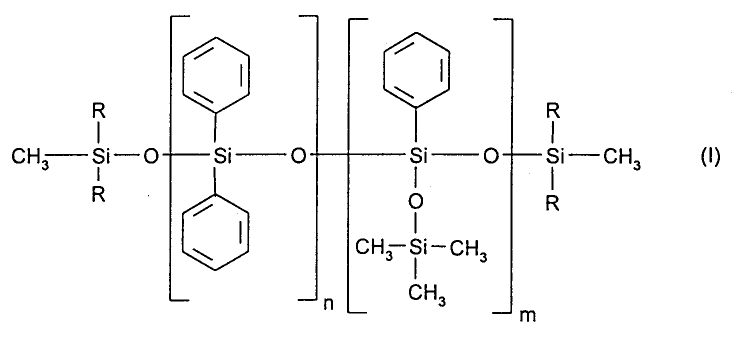 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.

De préférence, R est un radical méthyle, éthyle, propyle, isopropyle, décyle, dodécyle ou octadécyle, ou encore un radical phényle, tolyle, benzyle ou phénéthyle.
Parmi ces huiles phénylées, on peut citer l'huile Belsil PDM1000 de Wacker, les huiles DC556 ou SF558 de Dow Corning, l'huile Abil AV8853 de Goldschmidt ou l'huile Silbione 70633V30 de Rhône Poulenc.
La composition selon l'invention peut comprendre 1-35% en poids, de préférence 20-30%, d'huiles siliconées phénylées.

La phase grasse peut comprendre, en plus des huiles ci-dessus mentionnées, les corps gras usuellement utilisés dans le domaine d'application envisagé. Parmi ceux-ci, on peut citer les corps gras siliconés tels que les huiles, les corps gras pâteux, les gommes et les cires de silicone, ainsi que les corps gras non siliconés tels que les huiles, les pâteux et les cires végétales, minérales, animales et/ou synthétiques.
Parmi les corps gras siliconés, on peut citer les polydiméthylsiloxanes (PDMS) et les alkyldiméthicones, ainsi que les silicones modifiées par des groupements aliphatiques et/ou aromatiques, éventuellement fluorés, ou par des groupements fonctionnels tels que des groupements hydroxyles, thiols et/ou amines.
Parmi les corps gras non siliconés, on peut citer l'huile de paraffine, de vaseline, le perhydrosqualène, l'huile d'Arara, d'amande douce, de calophyllum, de palme, de ricin, d'avocat, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides; des huiles hydrogénées concrètes à 25°C; des lanolines; des esters gras concrets à 25°C; la cire d'abeilles; les cires végétales telles que la cire de Carnauba, de Candellila, d'Ourricury, du Japon ou les cires de fibres de lièges ou de canne à sucre; les cires minérales, par exemple de paraffine, de lignite ou les cires microcristallines ou les ozokérites; les cires synthétiques, parmi lesquelles les cires de polyéthylène et les cires obtenues par synthèse de Fischer-Tropsch.
Ces corps gras peuvent en particulier être choisis de manière variée par l'homme du métier afin de préparer une composition ayant les propriétés souhaitées, par exemple en consistance ou en texture.
En particulier, la composition selon l'invention peut comprendre au moins une cire, de manière à assurer la résistance mécanique, lorsque la composition se présente sous la forme d'un stick.
Lorsqu'elle se présente sous la forme d'une pâte souple ou d'un produit coulé, la composition selon l'invention comprend une quantité moins importante de cire, par exemple de l'ordre de 2-15% en poids.
D'une manière générale, la composition peut comprendre 0,5-30% en poids d'au moins une cire hydrocarbonée et/ou siliconée, et de préférence 10-20% en poids de cire hydrocarbonée et 0-10% en poids de cire siliconée.

De plus, on a constaté que l'amélioration de la tenue de la composition selon l'invention, ainsi que son absence de migration et/ou de transfert, pouvait être particulièrement intéressante lorsque la composition comprenait moins de 20% en poids d'huile non volatile hydrocarbonée, de préférence moins de 5% en poids, voire pas d'huiles hydrocarbonées non volatiles du tout.

La composition selon l'invention peut comprendre une phase particulaire, généralement présente à raison de 0-35% en poids, de préférence 5-25% en poids, et qui peut comprendre des pigments et/ou des nacres et/ou des charges habituellement utilisés dans les compositions cosmétiques.
Par pigments, il faut comprendre des particules blanches ou colorées, minérales ou organiques, insolubles dans la cire et la silicone volatile, destinées à colorer et/ou opacifier la composition. Par charges, il faut comprendre des particules incolores ou blanches, minérales ou de synthèse, lamellaires ou non lamellaires, destinées à donner du corps ou de la rigidité à la composition et/ou de la douceur, de la matité et de l'uniformité au maquillage. Par nacres, il faut comprendre des particules irisées qui réfléchissent la lumière.
Les pigments peuvent être présents dans la composition à raison de 0-15% en poids de la composition finale, et de préférence à raison de 8-10%. Ils peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium.
Les nacres peuvent être présentes dans la composition à raison de 0-20% en poids, de préférence à un taux élevé de l'ordre de 8-15% en poids. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.
Les charges, qui peuvent être présentes à raison de 0-30% en poids, de préférence 5-15%, dans la composition, peuvent être minérales ou de synthèse, lamellaires ou non lamellaires. On peut citer le talc, le mica, la silice, le kaolin, les poudres de Nylon et de polyéthylène, le Téflon, l'amidon, le micatitane, le nitrure de bore, les microsphères telles que l'Expancel (Nobel Industrie), le polytrap (Dow Corning) et les microbilles de résine de silicone (Tospearls de Toshiba).

La composition peut comprendre en outre tout additif usuellement utilisé dans le domaine cosmétique, tel que des antioxydants, des parfums, des huiles essentielles, des conservateurs, des actifs cosmétiques, des vitamines, des acides gras essentiels, des sphingolipides, des composés auto-bronzants tels que la DHA, des filtres solaires, des tensioactifs, des polymères liposolubles notamment hydrocarbonés, tels que le polybutène, les polyalkylènes, les polyacrylates et les polymères siliconés compatibles avec les corps gras. Ces additifs peuvent être présents dans la composition à raison de 0-10% en poids.
Bien entendu l'homme du métier veillera à choisir les éventuels composés complémentaires, et/ou leur quantité, de telle manière que les propriétés avantageuses de la composition selon l'invention ne soient pas, ou substantiellement pas, altérées par l'adjonction envisagée.

Les procédés de fabrication des compositions selon l'invention ne différent en rien des procédés classiquement utilisés en cosmétique et parfaitement connus de l'homme de l'art. Ces procédés consistent dans le mélange des différents constituants de la composition, préalablement chauffés, puis à leur coulage selon la forme souhaitée.

Les compositions selon l'invention peuvent se présenter notamment sous la forme de stick ou bâton, ou sous la forme de pâte souple ou coulée, voire sous la forme d'un liquide huileux, éventuellement gélifié.
Les compositions selon l'invention peuvent se présenter sous la forme d'un produit cosmétique et notamment sous la forme d'un produit de maquillage de la peau, en particulier un fond de teint, un fard à joues ou à paupières, ou un rouge à lèvres.
Elles peuvent également se présenter sous forme non colorée, contenant éventuellement des actifs cosmétiques. Elles peuvent alors notamment être utilisées comme base de soin pour les lèvres ou base fixante à appliquer sur un rouge à lèvres classique. La base fixante forme alors un film protecteur sur le film de rouge, en limite le transfert et la migration, et permet d'augmenter ainsi sa tenue.
Les compositions peuvent également se présenter sous la forme d'une composition dermatologique ou de soin de la peau, ou sous forme d'une composition solaire ou autobronzante.

L'invention est illustrée plus en détail dans les exemples suivants.

### Exemple 1

On prépare un bâton de rouge à lèvres ayant la composition suivante:

| | |
|---|---|
| . cyclopentadiméthylsiloxane | 35 g |
| . polyphénylméthylsiloxane (DC556 Fluid de Dow Corning) | 30 g |
| . cires de silicone | 10 g |
| . cires hydrocarbonées (notamment polyéthylène) | 10 g |
| . pigments | 10 g |
| . charges (notamment poudre de Nylon) | 5 g |

On prépare la composition de manière usuelle, en chauffant les corps gras, sauf les huiles volatiles, à 95°C et en les mélangeant. On ajoute ensuite les pigments et les charges, et, à 60°C, les huiles volatiles. On mélange le tout à l'aide d'une turbine Moritz à la vitesse de 3000 tr/min. On peut alors couler ledit mélange dans des moules adéquats.
On obtient ainsi un bâton de rouge à lèvres, de texture agréable, qui s'étale bien et s'applique uniformément. Le film est confortable à porter dans le temps et ne migre pas.

On applique cette composition sur la partie gauche des lèvres de plusieurs personnes. Pour comparaison, on applique sur la partie droite desdites lèvres, la même composition dans laquelle l'huile de silicone phénylée est remplacée par une huile hydrocarbonée d'origine végétale. On laisse sécher les rouges à lèvres à température ambiante pendant 5 minutes, puis on applique la totalité des lèvres sur une feuille de papier.
On constate sur la totalité des feuilles de papier, une trace plus importante de rouge laissée par la composition selon l'art antérieur. La composition selon l'invention laisse sur la feuille une trace très faible, à peine perceptible.

### Exemple 2

On prépare une pâte souple ayant la composition suivante :

| | |
|---|---|
| . cyclopentadiméthylsiloxane | 40 g |
| . polyphénylméthylsiloxane (DC556 Fluid de Dow Corning) | 20 g |
| . cire de silicone | 10 g |
| . cire de polyéthylène | 10 g |
| . pigments | 10 g |
| . charges (notamment poudre de Nylon) | 5 g |

On prépare la composition de manière usuelle, en chauffant les corps gras, sauf les huiles volatiles, à 95°C et en les mélangeant. On ajoute ensuite les pigments et les charges, et, à 60°C, les huiles volatiles. On mélange le tout à l'aide d'une turbine Moritz à la vitesse de 3000 tr/min. On peut alors couler ledit mélange dans des conditionnements adéquats. On obtient ainsi une pâte souple, de texture agréable, qui s'étale bien et s'applique uniformément. Le film est confortable à porter dans le temps et ne migre pas.

### Exemple 3

On prépare une base fixante pour rouge à lèvres ayant la composition suivante:

| | |
|---|---|
| . cyclotétradiméthylsiloxane | 35 g |
| . polyphénylméthylsiloxane (DC556 Fluid de Dow Corning) | 30 g |
| . cires de silicone | 10 g |
| . cires hydrocarbonées (notamment polyéthylène) | 10 g |
| . charges (notamment poudre de Nylon) | 5 g |

On prépare la composition selon l'exemple 1.

On obtient une base fixante sous forme de stick. Cette base est de texture agréable et s'applique aisément sur un film de rouge à lèvres classique.

## Revendications

1. Utilisation de l'association d'une huile volatile et d'une huile siliconée phénylée dans une composition comprenant une phase grasse afin de diminuer le transfert et/ou la migration de ladite composition.

2. Utilisation de l'association d'une huile volatile et d'une huile siliconée phénylée dans une composition comprenant une phase grasse afin d'améliorer la tenue de ladite composition.

3. Utilisation selon l'une des revendications précédentes, dans une composition comprenant moins de 20% d'huile hydrocarbonée non volatile.

4. Utilisation selon l'une des revendications précédentes, dans une composition comprenant moins de 5% d'huile hydrocarbonée non volatile.

5. Utilisation selon l'une des revendications précédentes, dans une composition ne comprenant pas d'huile hydrocarbonée non volatile.

6. Utilisation selon l'une des revendications précédentes, dans laquelle l'huile volatile est choisie parmi les huiles hydrocarbonées ou les huiles siliconées, cycliques ou linéaires, seules ou en mélange.

7. Utilisation selon l'une des revendications précédentes, dans laquelle l'huile volatile est choisie parmi la cyclotétradiméthylsiloxane, la cyclopentadiméthylsiloxane, la cyclohexadiméthylsiloxane, le méthylhexyldiméthylsiloxane, les isoparaffines, et leurs mélanges.

8. Utilisation selon l'une des revendications précédentes, dans laquelle l'huile volatile est présente à raison de 8-70% en poids, de préférence 30-60%, dans la composition.

9. Utilisation selon l'une des revendications précédentes, dans laquelle l'huile siliconée phénylée est choisie parmi les huiles de formule (I) et leurs mélanges, : 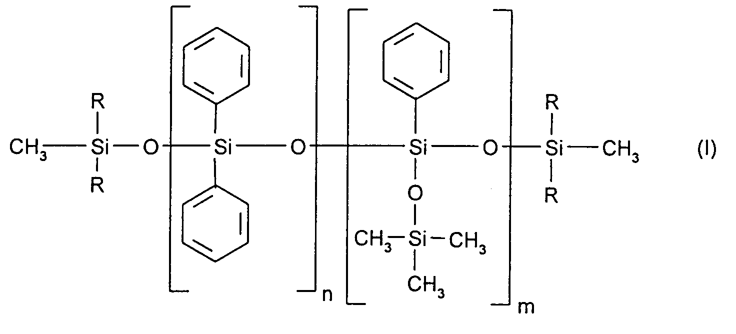 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.

10. Utilisation selon la revendication 9, dans laquelle l'huile siliconée phénylée est choisie parmi les huiles de formule (I) pour lesquelles R est un radical méthyle, éthyle, propyle, isopropyle, décyle, dodécyle, octadécyle, phényle, tolyle, benzyle ou phénéthyle.

11. Utilisation selon l'une des revendications précédentes, dans laquelle l'huile siliconée phénylée est présente à raison de 1-35% en poids, de préférence 20-30%, dans la composition.

12. Utilisation selon l'une des revendications précédentes, dans laquelle la phase grasse comprend en outre 0,5-30% en poids de cire hydrocarbonée et/ou siliconée.

13. Utilisation selon l'une des revendications précédentes dans une composition se présentant sous la forme de stick ou bâton, sous la forme de pâte souple ou coulée, ou sous la forme d'un liquide huileux, éventuellement gélifié.

14. Utilisation selon l'une des revendications précédentes dans une composition cosmétique ou dermatologique.

15. Utilisation selon l'une des revendications précédentes dans une composition de maquillage, notamment de fond de teint, de fard à joues ou à paupières, ou de rouge à lèvres; dans une base de soin ou une base fixante pour les lèvres; dans un produit de soin de la peau ou dans une composition solaire ou autobronzante.

16. Composition comprenant, dans une phase grasse, une huile volatile, une huile siliconée phénylée et moins de 20% en poids d'huile hydrocarbonée non volatile.

17. Composition selon la revendication 16, dans laquelle l'huile hydrocarbonée non volatile est présente à raison de moins de 5% en poids.

18. Composition selon l'une des revendications 16 à 17, dans laquelle l'huile volatile est choisie parmi les huiles hydrocarbonées ou les huiles siliconées, cycliques ou linéaires, seules ou en mélange.

19. Composition selon l'une des revendications 16 à 18, dans laquelle l'huile volatile est choisie parmi la cyclotétradiméthylsiloxane, la cyclopentadiméthylsiloxane, la cyclohexadiméthylsiloxane, le méthylhexyldiméthylsiloxane, les isoparaffines, et leurs mélanges.

20. Composition selon l'une des revendications 16 à 19, dans laquelle l'huile volatile est présente à raison de 8-70% en poids, de préférence 30-60%, dans la composition.

21. Composition selon l'une des revendications 16 à 20, dans laquelle l'huile siliconée phénylée est choisie parmi les huiles de formule (I) et leurs mélanges : 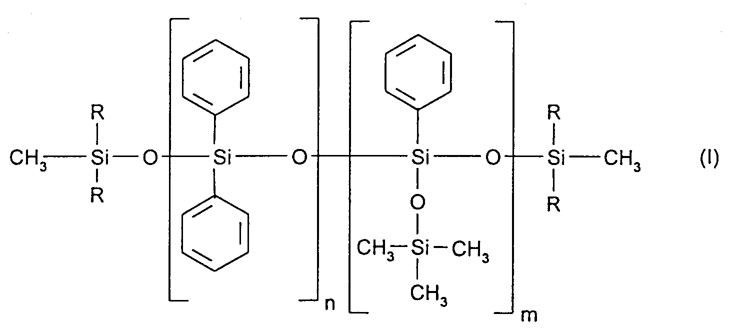 dans laquelle
. R est un radical alkyle en C1-C30, un radical aryle ou un radical aralkyle,
. n est un nombre entier compris entre 0 et 100,
. m est un nombre entier compris entre 0 et 100, sous réserve que la somme m+n est comprise entre 1 et 100.

22. Composition selon la revendication 21, dans laquelle l'huile siliconée phénylée est choisie parmi les huiles de formule (I) pour lesquelles R est un radical méthyle, éthyle, propyle, isopropyle, décyle, dodécyle, octadécyle, phényle, tolyle, benzyle ou phénéthyle.

23. Composition selon l'une des revendications 16 à 22, dans laquelle l'huile siliconée phénylée est présente à raison de 1-35% en poids, de préférence 20-30%, dans la composition.

24. Composition selon l'une des revendications 16 à 23, dans laquelle la phase grasse comprend en outre 0,5-30% en poids de cire hydrocarbonée et/ou siliconée, de préférence 10-20% en poids de cire hydrocarbonée et 0-10% en poids de cire siliconée.

25. Composition selon l'une des revendications 16 à 24, comprenant en outre une phase particulaire présente à raison de 0-35% en poids, de préférence 5-25% en poids.

26. Composition selon l'une des revendications 16 à 25 se présentant sous la forme de stick ou bâton, sous la forme de pâte souple ou coulée, ou sous la forme d'un liquide huileux, éventuellement gélifié.

27. Composition selon l'une des revendications 16 à 26 se présentant sous la forme d'une composition cosmétique ou dermatologique, comprenant un support cosmétiquement ou dermatologiquement acceptable.

28. Composition selon l'une des revendications 16 à 27 se présentant sous la forme d'une composition de maquillage, notamment un fond de teint, un fard à joues ou à paupières, ou un rouge à lèvres; d'une base de soin ou d'une base fixante pour les lèvres; d'un produit de soin de la peau ou d'une composition solaire ou autobronzante.

29. Procédé pour limiter, diminuer et/ou empêcher le transfert d'une composition de maquillage ou de soin de la peau, des muqueuses et/ou des semi-muqueuses, notamment un rouge à lèvres ou un fond de teint, consistant à introduire dans ladite composition une phase grasse comprenant une huile volatile, une huile siliconée phénylée et moins de 20% en poids d'huile hydrocarbonée non volatile.

## Claims

1. Use of the combination of a volatile oil and a phenylsilicone oil in a composition comprising a fatty phase in order to decrease the transfer and/or migration of the said composition.

2. Use of the combination of a volatile oil and a phenylsilicone oil in a composition comprising a fatty phase in order to improve the staying power of the said composition.

3. Use according to either of the preceding claims, in a composition comprising less than 20 % of non-volatile hydrocarbon oil.

4. Use according to one of the preceding claims, in a composition comprising less than 5 % of non-volatile hydrocarbon oil.

5. Use according to one of the preceding claims, in a composition comprising no non-volatile hydrocarbon oil.

6. Use according to one of the preceding claims, in which the volatile oil is chosen from hydrocarbon oils and silicone oils, which may be linear or cyclic, alone or as a mixture.

7. Use according to one of the preceding claims, in which the volatile oil is chosen from cyclotetradimethylsiloxane, cyclopentadimethylsiloxane, cyclohexadimethylsiloxane, methylhexyldimethylsiloxane and isoparaffins, and mixtures thereof.

8. Use according to one of the preceding claims, in which the volatile oil is present in a proportion of 8-70 % by weight, preferably 30-60 %, in the composition.

9. Use according to one of the preceding claims, in which the phenylsilicone oil is chosen from the oils of formula (I), and mixtures thereof: 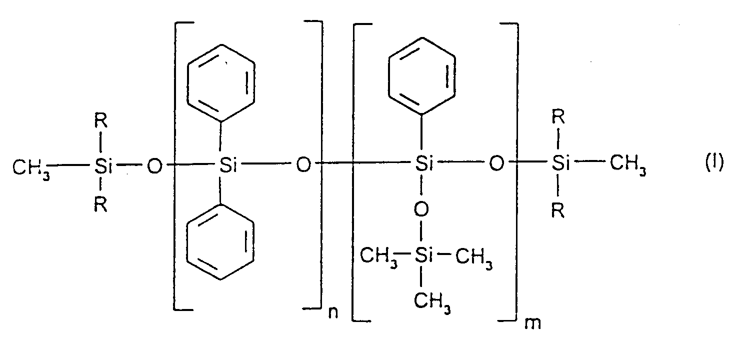 in which
• R is a C1-C30 alkyl radical, an aryl radical or an aralkyl radical,
• n is an integer between 0 and 100,
• m is an integer between 0 and 100, with the proviso that the sum m+n is between 1 and 100.

10. Use according to Claim 9, in which the phenylsilicone oil is chosen from the oils of formula (I) for which R is a methyl, ethyl, propyl, isopropyl, decyl, dodecyl, octadecyl, phenyl, tolyl, benzyl or phenethyl radical.

11. Use according to one of the preceding claims, in which the phenylsilicone oil is present in a proportion of 1-35 % by weight, preferably 20-30 %, in the composition.

12. Use according to one of the preceding claims, in which the fatty phase also comprises 0.5-30 % by weight of hydrocarbon wax and/or silicone wax.

13. Use according to one of the preceding claims, in a composition in the form of a stick or tube, in the form of a soft or cast paste or in the form of an oily liquid, which is optionally gelled.

14. Use according to one of the preceding claims, in a cosmetic or dermatological composition.

15. Use according to one of the preceding claims, in a make-up composition, in particular a foundation, blusher, eyeshadow or lipstick composition; in a care base or a fixing base for the lips; in a skincare product or in an antisun or self-tanning composition.

16. Composition comprising, in a fatty phase, a volatile oil, a phenylsilicone oil and less than 20 % by weight of non-volatile hydrocarbon oil.

17. Composition according to Claim 16, in which the non-volatile hydrocarbon oil is present in a proportion of less than 5 % by weight.

18. Composition according to either of Claims 16 and 17, in which the volatile oil is chosen from hydrocarbon oils and silicone oils, which may be linear or cyclic, alone or as a mixture.

19. Composition according to one of Claims 16 to 18, in which the volatile oil is chosen from cyclotetradimethylsiloxane, cyclopentadimethylsiloxane, cyclohexadimethylsiloxane, methylhexyldimethylsiloxane and iso-paraffins, and mixtures thereof.

20. Composition according to one of Claims 16 to 19, in which the volatile oil is present in a proportion of 8-70 % by weight, preferably 30-60 %, in the composition.

21. Composition according to one of Claims 16 to 20, in which the phenylsilicone oil is chosen from the oils of formula (I), and mixtures thereof: 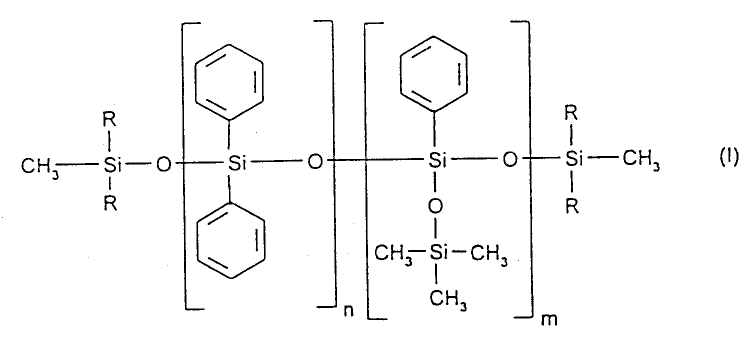 in which
• R is a C1-C30 alkyl radical, an aryl radical or an aralkyl radical,
• n is an integer between 0 and 100,
• m is an integer between 0 and 100, with the proviso that the sum m+n is between 1 and 100.

22. Composition according to Claim 21, in which the phenylsilicone oil is chosen from the oils of formula (I) for which R is a methyl, ethyl, propyl, isopropyl, decyl, dodecyl, octadecyl, phenyl, tolyl, benzyl or phenethyl radical.

23. Composition according to one of Claims 16 to 22, in which the phenylsilicone oil is present in a proportion of 1-35 % by weight, preferably 20-30 %, in the composition.

24. Composition according to one of Claims 16 to 23, in which the fatty phase also comprises 0.5-30 % by weight of hydrocarbon wax and/or silicone wax, preferably 10-20 % by weight of hydrocarbon wax and 0-10 % by weight of silicone wax.

25. Composition according to one of Claims 16 to 24, also comprising a particulate phase present in a proportion of 0-35 % by weight, preferably 5-25 % by weight.

26. Composition according to one of Claims 16 to 25, which is in the form of a stick or tube, in the form of a soft or cast paste or in the form of an oily liquid, which is optionally gelled.

27. Composition according to one of Claims 16 to 26, which is in the form of a cosmetic or dermatological composition comprising a cosmetically or dermatologically acceptable support.

28. Composition according to one of Claims 16 to 27, which is in the form of a make-up composition, in particular a foundation, blusher, eyeshadow or lipstick composition; of a care base or a fixing base for the lips; of a skincare product or of an antisun or self-tanning composition.

29. Process for limiting, reducing and/or preventing the transfer of a make-up or care composition for the face, mucous membranes and/or semi-mucous membranes, in particular a lipstick or a foundation, this process consisting in introducing into the said composition a fatty phase comprising a volatile oil, a phenylsilicone oil and less than 20 % by weight of non-volatile hydrocarbon oil.

## Patentansprüche

1. Verwendung der Kombination eines flüchtigen Öls und eines phenylgruppenhaltigen Siliconöls in einer Zusammensetzung, die eine Fettphase aufweist, um das Abfärben und/oder die Migration der Zusammensetzung zu vermindern.

2. Verwendung der Kombination eines flüchtigen Öls und eines phenylgruppenhaltigen Siliconöls in einer Zusammensetzung, die eine Fettphase aufweist, um die Haltbarkeit der Zusammensetzung zu verbessern.

3. Verwendung nach einem der vorhergehenden Ansprüche in einer Zusammensetzung, die weniger als 20 % nichtflüchtiges Kohlenwasserstofföl enthält.

4. Verwendung nach einem der vorhergehenden Ansprüche in einer Zusammensetzung, die weniger als 5 % nichtflüchtiges Kohlenwasserstofföl enthält.

5. Verwendung nach einem der vorhergehenden Ansprüche in einer Zusammensetzung, die kein nichtflüchtiges Kohlenwasserstofföl enthält.

6. Verwendung nach einem der vorhergehenden Ansprüche, wobei das flüchtige Öl unter den Kohlenwasserstofföllen oder den cyclischen oder geradkettigen Siliconölen oder deren Gemischen ausgewählt ist.

7. Verwendung nach einem der vorhergehenden Ansprüche, wobei das flüchtige Öl unter Cyclotetradimethylsiloxan, Cyclopentadimethylsiloxan, Cyclohexadimethylsiloxan, Methylhexyldimethylsiloxan, den Isoparaffinen und deren Gemischen ausgewählt ist.

8. Verwendung nach einem der Vorhergehenden Ansprüche, wobei das flüchtige Öl in einem Mengenanteil von 8 bis 70 Gew.-% und vorzugsweise 30 bis 60 % in der Zusammensetzung vorliegt.

9. Verwendung nach einem der vorhergehenden Ansprüche, wobei das phenylgruppenhaltige Siliconöl unter den Ölen der Formel (I) und deren Gemischen ausgewählt ist: 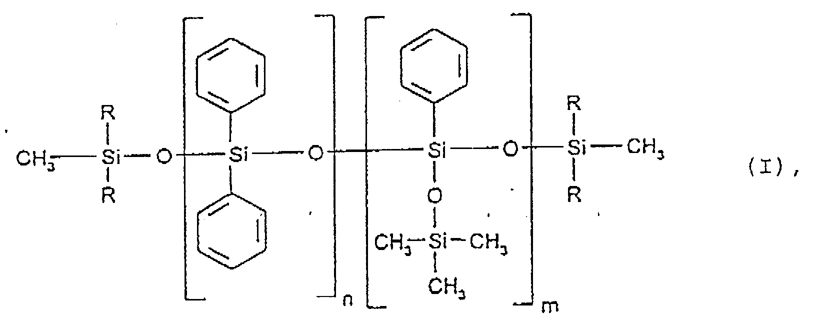 worin bedeuten:
R C₁₋₃₀-Alkyl, Aryl oder Aralkyl,
n eine ganze Zahl im Bereich von Null bis 100,
m eine ganze Zahl im Bereich von Null bis 100,
mit der Maßgabe, daß die Summe m+n im Bereich von 1 bis 100 liegt.

10. Verwendung nach Anspruch 9, wobei das phenylgruppenhaltige Siliconöl unter den Ölen der Formel (I) ausgewählt ist, wobei R Methyl, Ethyl, Propyl, Isopropyl, Decyl, Dodecyl, Octadecyl, Phenyl, Tolyl, Benzyl oder Phenethyl bedeutet.

11. Verwendung nach einem der vorhergehenden Ansprüche wobei das phenylgruppenhaltige Siliconöl in einem Mengenanteil von 1 bis 35 Gew.-% und vorzugsweise von 20 bis 30 Gew.-% in der Zusammensetzung vorliegt.

12. Verwendung nach einem der vorhergehenden Ansprüche, wobei die Fettphase ferner 0,5 bis 30 Gew.-% Kohlenwasserstoffwachs und/oder Siliconwachs enthält.

13. Verwendung nach einem der vorhergehenden Ansprüche in einer Zusammensetzung, die in Form eines Sticks oder Stiftes, in Form einer weichen oder gegossenen Paste oder in Form einer gegebenenfalls gelförmigen öligen Flüssigkeit vorliegt.

14. Verwendung nach einem der vorhergehenden Ansprüche in einer kosmetischen oder dermatologischen Zusammensetzung.

15. Verwendung nach einem der vorhergehenden Ansprüche in einer Zusammensetzung zum Schminken, insbesondere einem Make-up, Wangenrouge, Lidschatten, Lippenstift, einer Pflegegrundlage oder einem Mittel zur Fixierung für die Lippen, einem Produkt zur Pflege der Haut oder einem Sonnenschutzmittel oder Selbstbräunungsmittel.

16. Zusammensetzung, die in einer Fettphase ein flüchtiges Öl, ein phenylgruppenhaltiges Siliconöl und weniger als 20 Gew.-% nichtflüchtiges Kohlenwasserstofföl enthält.

17. Zusammensetzung nach Anspruch 16, wobei das nichtflüchtige Kohlenwasserstofföl in einem Mengenanteil von weniger als 5 Gew.-% vorliegt.

18. Zusammensetzung nach einem der Ansprüche 16 bis 17, wobei das flüchtige Öl unter den Kohlenwasserstoffölen oder cyclischen oder geradkettigen Siliconölen oder deren Gemischen ausgewählt ist.

19. Zusammensetzung nach einem der Ansprüche 16 bis 18, wobei das flüchtige Öl unter Cyclotetradimethylsiloxan, Cyclopentadimethylsiloxan, Cyclohexadimethylsiloxan, Methylhexyldimethylsiloxan, den Isoparaffinen und deren Gemischen ausgewählt ist.

20. Zusammensetzung nach einem der Ansprüche 16 bis 19, wobei daa flüchtige Öl in einem Mengenanteil von 8 bis 70 Gew.-% und vorzugsweise 30 bis 60 Gew.-% in der Zusammensetzung vorliegt.

21. Zusammensetzung nach einem der Ansprüche 16 bis 20, wobei das phenylgruppenhaltige Siliconöl unter den Ölen der Formel (I) und deren Gemischen ausgewählt ist: 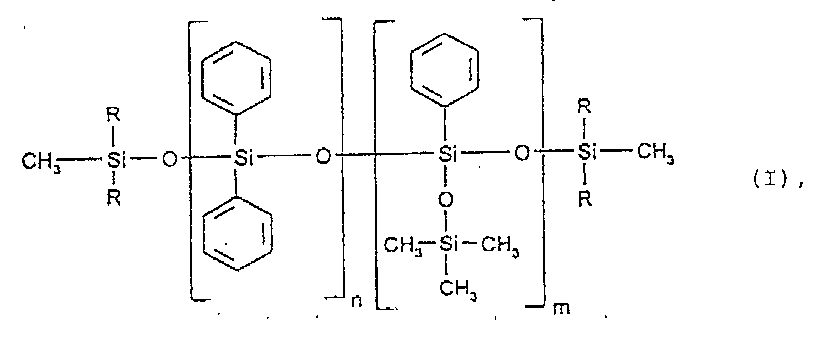 worin bedeuten:
R C₁₋₃₀-Alkyl, Aryl oder Aralkyl,
n eine ganze Zahl im Bereich von Null bis 100,
m eine ganze Zahl im Bereich von Null bis 100,
mit der Maßgabe, daß die Summe m+n im Bereich von 1 bis 100 liegt.

22. Zusammensetzung nach Anspruch 21, wobei das phenylgruppenhaltige Siliconöl unter den Ölen der Formel (I) ausgewählt ist, wobei R Methyl, Ethyl, Propyl, Isopropyl, Decyl, Dodecyl, Octadecyl, Phenyl, Tolyl, Benzyl oder Phenethyl bedeutet.

23. Zusammensetzung nach einem der Ansprüche 16 bis 22, wobei das phenylgruppenhaltige Siliconöl in einem Mengenanteil von 1 bis 35 Gew.-% und vorzugsweise von 20 bis 30 Gew.-% in der Zusammensetzung vorliegt.

24. Zusammensetzung nach einem der Ansprüche 16 bis 23, wobei die Fettphase ferner 0,5 bis 30 Gew.-% Kohlenwasserstoffwachs und/oder Siliconwachs und vorzugsweise 10 bis 20 Gew.-% Kohlenwasserstoffwachs und 0 bis 10 Gew.-% Siliconwachs enthält.

25. Zusammensetzung nach einem der Ansprüche 16 bis 24, die ferner eine spezielle Phase enthält, die in einem Mengenanteil von 0 bis 35 Gew.-% und vorzugsweise von 5 bis 25 Gew.-% vorliegt.

26. Zusammensetzung nach einem der Ansprüche 16 bis 25, die in Form eines Sticks oder Stiftes, in Form einer weichen oder gegossenen Paste oder in Form einer gegebenenfalls gelförmigen öligen Flüssigkeit vorliegt.

27. Zusammensetzung nach einem der Ansprüche 16 bis 26, die in Form einer kosmetischen oder dermatologischen Zusammensetzung vorliegt und einen kosmetisch oder dermatologisch akzeptablen Träger enthält.

28. Zusammensetzung nach einem der Ansprüche 16 bis 27, die in Form einer Zusammensetzung zum Schminken, insbesondere als Make-up, Wangenrouge, Lidschatten oder Lippenstift, Pflegegrundlage oder Mittel zur Fixierung für die Lippen, als Produkt zur Pflege der Haut oder als Sonnenschutzmittel oder Selbstbräunungsmittel vorliegt.

29. Verfahren, um das Abfärben einer Zusammensetzung zum Schminken oder zur Pflege der Haut, der Schleimhäute und/oder der Semischleimhäute zu begrenzen, zu vermindern und/oder zu verhindern, insbesondere eines Lippenstiftes oder Make-ups, das darin besteht, in die Zusammensetzung eine Fettphase zu geben, die ein flüchtiges Öl, ein phenylgruppenhaltiges Siliconöl und weniger als 20 Gew.-% nichtflüchtiges Kohlenwasserstofföl enthält.
